# EUROPÄISCHE PATENTSCHRIFT

(11) **EP 4 076 384 B1**
(45) Veröffentlichungstag und Bekanntmachung des Hinweises auf die Patenterteilung: **13.11.2024**
(21) Anmeldenummer: 20835795.4
(22) Anmeldetag: 18.12.2020
(51) Int. Cl.: A61K 9/00, A61K 9/107, B01J 13/00

(54) **VERFAHREN ZUR HERSTELLUNG VON ÖL-IN-WASSER-EMULSIONEN**
METHOD FOR MANUFACTURING OIL-IN-WATER EMULSIONS
PROCÉDÉ DE FABRICATION D'ÉMULSIONS HUILE DANS EAU

(30) Priorität: 20.12.2019 EP 19218741
(43) Veröffentlichungstag der Anmeldung: 26.10.2022
(73) Patentinhaber: Fresenius Kabi Austria GmbH, 8055 Graz (AT)
(72) Erfinder: JUNG, Andreas, Heinrich, 8055 Graz (AT); KÖTH, Christoph, 8055 Graz (AT); STADLER, Peter, Gerhard, 8055 Graz (AT)
(74) Vertreter: Fresenius Kabi Deutschland GmbH
(86) Internationale Anmeldenummer: PCT/EP2020/086956
(87) Internationale Veröffentlichungsnummer: WO 2021/123117

(56) Entgegenhaltungen:
- US-A- 5 637 625
- US-A1- 2007 014 805
- US-A1- 2014 363 474
- US-A1- 2018 036 237

## Beschreibung

### GEBIET DER ERFINDUNG

Die vorliegende Erfindung betrifft die Herstellung von Öl-in-Wasser-Emulsionen, die nach dem erfindungsgemäßen Verfahren hergestellten Emulsionen sowie deren Verwendung als Medikament oder in der Bereitstellung parenteraler Ernährung.

### HINTERGRUND DER ERFINDUNG

Emulsionen sind disperse Systeme zweier nicht mischbarer Flüssigkeiten. Dabei wird zwischen der inneren oder dispergierten Phase, die in diskrete Tröpfchen zerteilt ist, und der äußeren Phase, dem Dispersionsmittel unterschieden.

Solche Systeme sind ohne weitere Zusätze nicht stabil, können aber beispielsweise durch den Zusatz von Emulgatoren stabilisiert werden. Emulgatoren gehören zu den grenzflächenaktiven Substanzen. Sie lagern sich an der Phasengrenze an, erleichtern die Bildung der Tröpfchen, die die innere Phase in der äußeren Phase bildet, und wirken der Phasentrennung entgegen.

Bei Öl-in-Wasser-Emulsionen liegen Öltröpfchen dispergiert in einer Wasserphase vor.

Öl-in-Wasser-Emulsionen werden für die unterschiedlichsten Zwecke hergestellt, z.B. als Emulsionen für den Einsatz in der parenteralen Ernährung oder als Basis von Emulsionen, die Arzneistoffe wie beispielsweise Propofol enthalten.

Öl-in-Wasser-Emulsionen zur parenteralen Verabreichung müssen, zumindest wenn sie in größeren Volumina verabreicht werden, zudem eine dem Blut möglichst ähnliche Osmolalität aufweisen, d.h. ihnen muss ein Isotonisierungsmittel zugesetzt werden.

Ein Nachteil dieser Öl-in-Wasser-Emulsionen besteht darin, dass ihre Herstellung, insbesondere in der für die parenterale Verabreichung erforderlichen Güte, aufwendig und mit einer sehr hohen Ausschussrate behaftet ist. Pharmazeutische Öl-in-Wasser-Emulsionen zur parenteralen Anwendung werden standardmäßig in einem zweistufigen Prozess erzeugt, bei dem zunächst aus der Wasserphase (die das Isotonisierungsmittel enthält), einem Emulgator und der Ölphase eine Prä-Emulsion hergestellt wird, in der der Durchmesser der Öltröpfchen im Mikrometerbereich liegt. Das Herstellen der Prä-Emulsion kann beispielsweise mit Hilfe eines Rotor-Stator-Dispergators erfolgen.

Aus der Prä-Emulsion wird dann - beispielsweise durch einen mehrstufigen Hochdruckhomogenisierungsprozess - eine Emulsion gewonnen. Dabei werden die Öltröpfchen verkleinert, so dass diese Emulsion danach auch die Anforderungen an parenteral verabreichte Zubereitungen, u.a. hinsichtlich der Tröpfchengrößenverteilung erfüllt.

Bei Öl-in-Wasser-Emulsionen für die parenterale Verabreichung darf aus physiologischen Gründen (geschuldet der Anatomie und Größe der Blutgefäße) der mittlere Durchmesser der Öltröpfchen 0,5 µm nicht überschreiten.

Zusätzlich muss bei parenteral verabreichten Emulsionen der PFATs-Wert (der prozentuale Anteil von Öltropfen innerhalb der Ölphase einer Öl-in-Wasser-Emulsion mit einem Durchmesser von mehr als 5 µm) unter 0,05 % liegen (siehe USP 729).

Es kommt allerdings sehr häufig vor, dass dieser Grenzwert überschritten wird. In der Praxis findet man bei etwa 20 bis 30% der gefertigten Emulsionen einen PFATs-Wert von über 0,05 %. Diese Chargen müssen vernichtet werden, was enormen wirtschaftlichen Schaden verursacht.

Außerdem sind die derzeit standardmäßig praktizierten Verfahren sehr zeit- und energieaufwendig.

Aufgabe der vorliegenden Erfindung ist es daher, ein zeit- und energieeffizienteres Verfahren zur Herstellung von Öl-in-Wasser-Emulsionen bereitzustellen, das die zuverlässige und reproduzierbare Gewinnung von Emulsionen gestattet, die die hohen Anforderungen an parenteral verabreichte Zusammensetzungen erfüllen.

### ZUSAMMENFASSUNG DER ERFINDUNG

Diese Aufgabe wird überraschend dadurch gelöst, dass in dem erfindungsgemäßen Herstellungsverfahren für Öl-in-Wasser-Emulsionen große Teile der Wasserphase erst nach dem energieaufwendigen Homogenisieren der Prä-Emulsion zugegeben werden, d.h. es wird zunächst eine Emulsion mit geringerem Wassergehalt hergestellt, die danach "verdünnt" wird.

Die Erfindung betrifft also ein Verfahren zur Herstellung einer Öl-in-Wasser-Emulsion umfassend eine Wasserphase und 1 bis 40, bevorzugt 5 bis 30, am meisten bevorzugt 10 bis 30 % einer Ölphase bezogen auf das Gesamtgewicht der Emulsion, wobei das Verfahren die folgenden Schritte umfasst:
a) Bereitstellen einer Ölphase umfassend eines oder mehrere Öle und optional zumindest ein pharmazeutisch verträgliches Antioxidans und/oder zumindest einen pharmazeutisch verträglichen Co-Emulgator,
b) Bereitstellen einer Wasserphase 1 umfassend Wasser und optional zumindest einen pharmazeutisch verträglichen Co-Emulgator und/oder zumindest eine Substanz zur Einstellung des pH-Wertes und/oder zumindest ein pharmazeutisch verträgliches Konservierungsmittel und/oder zumindest ein pharmazeutisch verträgliches Isotonisierungsmittel, wobei das Isotonisierungsmittel in einer Konzentration von höchstens 18 %, vorzugsweise 15 %, insbesondere bevorzugt höchstens 14,3 %, bezogen auf das Gesamtgewicht der Wasserphase 1 vorliegt,
c) Herstellen einer Prä-Emulsion durch Mischen der Ölphase, bereitgestellt in Schritt a), mit der Wasserphase 1, bereitgestellt in Schritt b),
d) Herstellen einer ersten Emulsion durch Homogenisieren der Prä-Emulsion, bereitgestellt in Schritt c),
e) Bereitstellen einer Wasserphase 2 umfassend Wasser, und optional zumindest ein pharmazeutisch verträgliches Isotonisierungsmittel und/oder zumindest eine Substanz zur Einstellung des pH-Wertes und/oder zumindest ein pharmazeutisch verträgliches Konservierungsmittel,
f) Herstellen der Emulsion durch Mischen der ersten Emulsion, bereitgestellt in Schritt d) mit der Wasserphase 2, bereitgestellt in Schritt e) und
g) Sterilisieren der Emulsion, erhalten in Schritt f),

wobei die Emulsion vor oder nach dem Sterilisieren in einen geeigneten Behälter gefüllt wird,
wobei in Schritt a) und/oder in Schritt b) zumindest ein pharmazeutisch verträglicher Emulgator zugesetzt wird, und
wobei die Wasserphase 1, bereitgestellt in Schritt b) nicht mehr als 70 %, bevorzugt nicht mehr als 50 %, insbesondere bevorzugt nicht mehr 30 % und am meisten bevorzugt nicht mehr als 20 % der in der Emulsion enthaltenen Gesamtwassermenge bereitstellt.

Die Erfindung betrifft darüber hinaus die nach diesem Verfahren erhältliche Öl-in-Wasser-Emulsion und ihre Verwendung als Medikament oder in der Bereitstellung parenteraler Ernährung.

Die Erfindung betrifft außerdem die in Schritt d) erhaltene erste Emulsion.

Die Erfindung wird durch die Ansprüche definiert.

### DETAILLIERTE BESCHREIBUNG DER ERFINDUNG

Bei den bekannten Verfahren zur Herstellung von Öl-in-Wasser-Emulsionen wird zunächst aus der gesamten Wasserphase, der vorab ggf. zumindest ein Isotonisierungsmittel sowie ggf. zumindest ein Konservierungsmittel, ggf. zumindest ein Mittel zur pH-Wert-Einstellung und/oder weitere wasserlösliche Substanzen zugesetzt wurden, und der gesamten Ölphase, der vorab ggf. zumindest ein Antioxidans und ggf. weitere lipophile Bestandteile zugesetzt wurden, in Anwesenheit zumindest eines Emulgators, der vorab in Abhängigkeit von seiner chemischen Natur entweder der Öl- oder der Wasserphase zugesetzt wurde, durch intensives Mischen eine Prä-Emulsion hergestellt.

Diese Prä-Emulsion wird durch Homogenisieren - beispielsweise mit Hilfe von Hochdruckhomogenisatoren, Gegenstrahldispergatoren oder unter Einsatz von Ultraschall - in eine Emulsion überführt, in der die mittlere Größe der Öltröpfchen im Vergleich zur Prä-Emulsion erheblich verringert ist.

Diese Vorgänge sind zeit- und energieaufwendig und benötigen unter Umständen großvolumiges Gerät.

Es wurde nun überraschend gefunden, dass bei der Herstellung der Prä-Emulsion und bei der Gewinnung der Emulsion Teile der Wasserphase weggelassen und erst nach der Emulgierung zugefügt werden können, ohne dass die Qualität der Emulsionen beeinträchtigt wird, sofern die Konzentration des Isotonisierungsmittels in der Wasserphase 1 während des Emulgiervorganges 18 %, vorzugsweise 15 %, insbesondere bevorzugt höchstens 14,3 bezogen auf das Gesamtgewicht der Wasserphase 1, nicht überschreitet.

Das erfindungsgemäße Verfahren zur Herstellung von Öl-in-Wasser-Emulsionen umfassend eine Wasserphase und 1 bis 40 %, bevorzugt 5 bis 30 %, am meisten bevorzugt 10 bis 30 % einer Ölphase bezogen auf das Gesamtgewicht der Emulsion, umfasst die folgenden Schritte:
a) Bereitstellen einer Ölphase umfassend eines oder mehrere Öle und optional zumindest ein pharmazeutisch verträgliches Antioxidans und/oder zumindest einen pharmazeutisch verträglichen Co-Emulgator,
b) Bereitstellen einer Wasserphase 1 umfassend Wasser, optional zumindest einen pharmazeutisch verträglichen Co-Emulgator und/oder zumindest eine Substanz zur Einstellung des pH-Wertes und/oder zumindest ein pharmazeutisch verträgliches Konservierungsmittel und/oder zumindest ein pharmazeutisch verträgliches Isotonisierungsmittel, wobei das Isotonisierungsmittel in einer Konzentration von höchstens 18 %, vorzugsweise 15 %, insbesondere bevorzugt höchstens 14,3 %, bezogen auf das Gesamtgewicht der Wasserphase 1 vorliegt,
c) Herstellen einer Prä-Emulsion durch Mischen der Ölphase, bereitgestellt in Schritt a), mit der Wasserphase 1, bereitgestellt in Schritt b),
d) Herstellen einer ersten Emulsion durch Homogenisieren der Prä-Emulsion, bereitgestellt in Schritt c),
e) Bereitstellen einer Wasserphase 2 umfassend Wasser, optional zumindest ein pharmazeutisch verträgliches Isotonisierungsmittel und/oder zumindest eine Substanz zur Einstellung des pH-Wertes und/oder zumindest ein pharmazeutisch verträgliches Konservierungsmittel,
f) Herstellen der Emulsion durch Mischen der ersten Emulsion, bereitgestellt in Schritt d) mit der Wasserphase 2, bereitgestellt in Schritt e) und
g) Sterilisieren der Emulsion, erhalten in Schritt f),

wobei die Emulsion vor oder nach dem Sterilisieren in einen geeigneten Behälter gefüllt wird,
wobei in Schritt a) und/oder in Schritt b) zumindest ein pharmazeutisch verträglicher Emulgator zugesetzt wird, und
wobei die Wasserphase 1, bereitgestellt in Schritt b) nicht mehr als 70 %, bevorzugt nicht mehr als 50 %, insbesondere bevorzugt nicht mehr 30 % und am meisten bevorzugt nicht mehr als 20 % der in der Emulsion enthaltenen Gesamtwassermenge bereitstellt.

Die vorliegende Erfindung betrifft außerdem die nach diesem Verfahren hergestellten Öl-in-Wasser-Emulsionen sowie deren Verwendung als Medikament oder in der Bereitstellung parenteraler Ernährung.

Die vorliegende Erfindung betrifft zudem die in Schritt d) des Verfahrens erhaltenen Emulsionen, die vor ihrer erfindungsmäßen Weiterverarbeitung in den Schritten e), f) und g) verpackt, gelagert und transportiert werden können.

Außerdem betrifft die vorliegende Erfindung die in Schritt d) erhaltenen Emulsionen zur Verwendung in der Weiterverarbeitung gemäß den Schritten e), f) und g) des erfindungsgemäßen Verfahrens.

Das erfindungsgemäße Verfahren ist in mehrfacher Hinsicht vorteilhaft:
Erstens wird durch die Verringerung des Volumens der Wasserphase Kapazität im Homogenisierwerkzeug gespart, was verkürzte Homogenisierungszeiten und damit eine deutlich verbesserte Ausbeute/Effizienz bedingt. (So benötigt beispielsweise ein Hochdruckhomogenisator zur Homogenisierung von 1000 kg einer Prä-Emulsion etwa eine Stunde.)

Zweitens werden dadurch, dass in den energieaufwendigen Arbeitsschritten geringere Massen verarbeitet werden müssen, Zeit und Energie gespart. Neben der oben genannten Effizienzsteigerung beim Homogenisieren, wird aufgrund ihrer reduzierten Masse insbesondere das Erwärmen der Wasserphase energie- und zeiteffizienter.

Drittens besteht auch bei den Kessel- und Raumgrößen innerhalb der Produktionsanlagen erhebliches Einsparpotential.

Überraschenderweise liefert das erfindungsgemäße Verfahren außerdem Emulsionen besserer Qualität. Verbesserungen sind insbesondere hinsichtlich der Tröpfchengrößenverteilung festzustellen.

So ist bei den Emulsionen, die nach dem erfindungsgemäßen Verfahren hergestellt werden, die Tröpfchengrößenverteilung reproduzierbarer und enger.

Außerdem sind bei den nach dem erfindungsgemäßen Verfahren hergestellten Emulsionen die PFATs-Werte deutlich niedriger.

Die nach dem erfindungsgemäßen Verfahren hergestellten Emulsionen weisen vorzugsweise einen PFATs-Wert von unter 0,05 %, insbesondere bevorzugt von unter 0,04 %, stärker bevorzugt von unter 0,03 % und am meisten bevorzugt von unter 0,02 % auf.

Die nach dem erfindungsgemäßen Verfahren hergestellten Emulsionen weisen vorzugsweise einen durchschnittlichen PFATs-Wert von unter 0,035 %, besonders bevorzugt von unter 0,030 % und insbesondere bevorzugt von unter 0,025 % auf. Ganz besonders bevorzugt weisen die nach dem erfindungsgemäßen Verfahren hergestellten Emulsionen, stärker bevorzugt von unter 0,020 %, noch stärker bevorzugt von unter 0,015 % und am meisten bevorzugt von unter 0,010 % auf. Für die Bestimmung dieses durchschnittlichen Wertes ist auf eine Stichprobengröße von mindestens 10 abzustellen.

Bei der Herstellung nach den bekannten Standardverfahren findet man in der Praxis häufig PFATs-Werte von über 0,05 %. Überraschenderweise verringert das erfindungsgemäße Verfahren den PFATs-Wert auf weit unter 0,05 % und den durchschnittlichen PFAT5-Wert auf weit unter 0,035 %. Die vorliegende Erfindung betrifft darüber hinaus eine Anlage zur Herstellung einer Öl-in-Wasser-Emulsion, wobei die Öl-in-Wasser-Emulsion eine Wasserphase und 1 bis 40 %t, bevorzugt 5 bis 30 %, am meisten bevorzugt 10 bis 30 %, einer Ölphase bezogen auf das Gesamtgewicht der Emulsion umfasst, umfassend
a) einen ersten Kessel zum Bereitstellen einer Ölphase umfassend folgende Komponenten: ein oder mehrere Öle und optional zumindest ein pharmazeutisch verträgliches Antioxidans und/oder zumindest einen pharmazeutisch verträglichen Co-Emulgator sowie eine erste Einrichtung zum Mischen und/oder Dispergieren, vorzugsweise Rühren, der Komponenten in dem ersten Kessel,
b) einen zweiten Kessel zum Bereitstellen einer Wasserphase 1 umfassend folgende Komponenten: Wasser, optional zumindest einen pharmazeutisch verträglichen Co-Emulgator und/oder zumindest eine Substanz zur Einstellung des pH-Wertes und/oder zumindest ein pharmazeutisch verträgliches Konservierungsmittel und/oder zumindest ein pharmazeutisch verträgliches Isotonisierungsmittel, wobei das Isotonisierungsmittel in einer Konzentration von höchstens 18 %t, vorzugsweise höchstens 15 %, bezogen auf das Gesamtgewicht der Wasserphase 1 vorliegt, sowie eine zweite Einrichtung zum Mischen und/oder Dispergieren, vorzugsweise Rühren, der Komponenten in dem zweiten Kessel,
c)einen Tank zur Aufnahme der Komponenten aus dem ersten Kessel über einen ersten Sterilfilter und der Komponenten aus dem zweiten Kessel über einen zweiten Sterilfilter, wobei der Tank eine dritte Einrichtung zum Mischen und/oder Dispergieren aufweist, zum Herstellen einer Prä-Emulsion durch Mischen der Ölphase aus dem ersten Kessel und der Wasserphase 1 aus dem zweiten Kessel,
d) einen Homogenisator, vorzugsweise einen Hochdruckhomogenisator, zum Herstellen einer ersten Emulsion durch Homogenisieren der Prä-Emulsion,
e) einen Lagertank zum Bereitstellen einer Wasserphase 2 umfassend die folgenden Komponenten: Wasser und optional zumindest ein pharmazeutisch verträgliches Isotonisierungsmittel und/oder zumindest eine Substanz zur Einstellung des pH-Wertes und/oder zumindest ein pharmazeutisch verträgliches Konservierungsmittel sowie gegebenenfalls eine dritte Einrichtung zum Mischen, vorzugsweise Rühren, der Komponenten in dem Lagertank,
f) eine Einrichtung zum Überführen der Emulsion in den Lagertank zum Herstellen der Emulsion durch Mischen der ersten Emulsion aus dem Homogenisator und der Wasserphase 2 aus dem Lagertank und
g) eine Einrichtung zum Sterilisieren der Emulsion,
h) eine Einrichtung zum Abfüllen der Emulsion vor oder nach dem Sterilisieren in einen geeigneten Behälter,

wobei in dem ersten Kessel und/oder in dem zweiten Kessel zumindest ein pharmazeutisch verträglicher Emulgator zugesetzt wird, und
wobei die Wasserphase 1, bereitgestellt in dem zweiten Kessel, nicht mehr als 70 %, bevorzugt nicht mehr als 50 %, insbesondere bevorzugt nicht mehr 30 % und am meisten bevorzugt nicht mehr als 20 % der in der Emulsion enthaltenen Gesamtwassermenge bereitstellt.

Erfindungsgemäß kann in der Anlage zur Herstellung der Öl-in-Wasser-Emulsion der Tank c) identisch mit dem Kessel a) oder dem Kessel b) sein.

### Verwendung der erfindungsgemäß hergestellten Öl-in-Wasser-Emulsionen

Die nach dem erfindungsgemäßen Verfahren hergestellten Öl-in-Wasser-Emulsionen werden vorzugsweise parenteral, insbesondere bevorzugt intravenös, verabreicht und als Medikament oder in der Bereitstellung parenteraler Ernährung verwendet.

### Die Inhaltsstoffe

Wo die Konzentration der Inhaltstoffe in Prozent angegeben ist, bezieht sich diese Prozentangabe auf Massenanteile (Masse/Masse). So bedeutet "10 % Ölphase bezogen auf das Gesamtgewicht der Emulsion" so viel wie "10 g Ölphase pro 100 g Emulsion".

### Die Öle

Die nach dem erfindungsgemäßen Verfahren hergestellten Öl-in-Wasser-Emulsionen enthalten 1 bis 40 %, bevorzugt 5 bis 30 % am meisten bevorzugt 10 bis 30 %, beispielsweise 10 %, 20 % oder 30 % einer Ölphase bezogen auf das Gesamtgewicht der Emulsion.

Die Ölphase umfasst ein oder mehrere Öle ausgewählt aus der Gruppe bestehend aus Fischöl, Fischölextrakt oder Krillöl, mikrobiell erzeugten Ölen, Algen-Ölen, und Pflanzenölen wie Sojaöl, Sonnenblumenöl, Kokosöl, Olivenöl, Rapsöl, Erdnussöl, Palmöl, Sesamöl, Distelöl, Mandelöl, Leinöl oder Baumwollsamenöl.

Vorzugsweise umfasst die Ölphase Sojaöl, Sonnenblumenöl, Kokosöl, mittelkettige Triglyceride (MCT), Olivenöl, Rapsöl, Fischöl, Fischölextrakt, Krillöl oder Mischungen davon.

Insbesondere bevorzugt umfasst die Ölphase Sojaöl, MCT, Olivenöl, Fischöl oder Mischungen davon, beispielsweise Mischungen aus Sojaöl und MCT oder Mischungen aus Fischöl, Sojaöl, Olivenöl und MCT.

In einer besonders bevorzugten Ausführungsform umfasst die Ölphase 25 bis 35 %, vorzugsweise 30 %, Sojaöl, 25 bis 35 %, vorzugsweise 30 %, MCT, 20 bis 30 %, vorzugsweise 25 %, Olivenöl und 10 bis 20 %, vorzugsweise 15 %, Fischöl bezogen auf das Gesamtgewicht der Ölphase.

Unter "Fischöl" wird im Zusammenhang mit der vorliegenden Erfindung "gereinigtes Fischöl" und "gereinigtes Fischöl reich an n-3-Fettsäuren" gemäß dem Europäischen Arzneibuch 6.0 verstanden. Es enthält mindestens 9 % Docosahexaensäure (DHA) und mindestens 13 % Eicosapentaensäure (EPA) als Triglyceride bezogen auf das Gesamtgewicht des Fischöls.

Der Begriff "Fischölextrakt" bezieht sich auf Mischungen mit hohen EPA und DHA-Gehalten, die beispielsweise durch superkritische Flüssigextraktion und anschließende, z.B. chromatographische, Aufreinigung aus Fischöl gewonnen werden. Alternativ kann das Öl wie in US6750048 beschrieben extrahiert werden. Weitere Extraktions- und/oder Aufreinigungsmethoden sind in WO2001/076715 and WO2001/076385 beschrieben. Fischölextrakt enthält EPA und DHA in veresterter Form, beispielsweise in Form ihrer Triglyceride oder Ethylester.

Der Begriff "mittelkettige Triglyceride" bezieht sich auf die Triglyceride von Fettsäuren mit einer Kettenlänge von 6 bis 12 Kohlenstoffatomen, beispielsweise Caprylsäure, Capronsäure, Caprinsäure und Laurinsäure.

### Das Wasser

Da die nach dem erfindungsgemäßen Verfahren hergestellten Öl-in-Wasser-Emulsionen vorzugsweise parenteral verabreicht werden, ist das zur Bereitstellung der Wasserphasen 1 und 2 verwendete Wasser vorzugsweise Wasser für Injektionszwecke (WFI).

### Der Emulgator

Das erfindungsgemäße Verfahren umfasst die Zugabe zumindest eines Emulgators. Der Begriff "Emulgator" bezieht sich auf amphiphile Substanzen, die die Emulsion durch Reduktion der Grenzflächenspannung zwischen der Öl- und der Wasserphase stabilisieren.

Der Emulgator kann jeder pharmazeutisch verträgliche, zur Herstellung von Öl-in-Wasser-Emulsionen geeignete Emulgator sein. Geeignete Emulgatoren sind Lecithine, chemisch modifizierte Lecithine (z.B. hydrierte und/oder ethoxylierte Lecithine), Phospholipide, Sphingolipide, Sterole (z.B. Cholesterin sowie Derivate und Alkali- und Erdalkalisalze von Cholesterin, Stigmasterol), Gallensäuren und deren Salze (z.B. Natriumcholat, Natriumglycolcholat, Natriumtaurocholat), Blockpolymere und Block-Co-Polymere (z.B. Poloxamere wie Pluronic F68, F127 und Poloxamine wie Tetronic 1304), Polyglycerin-Ether, Polyglycerin-Ester, Ester von Zuckern mit Fettsäuren und/oder Fettalkoholen (z.B. Sucrosemonostearat, Glycerinmonooleat) und ethoxylierte Sorbitanfettsäureester (z.B. Tween 20, 40, 60, 80).

Sie werden in Konzentrationen von 0,1 bis 5 %, vorzugsweise 0,6 bis 3 % bezogen auf das Gesamtgewicht der Emulsion eingesetzt.

Vorzugsweise ist der Emulgator Lecithin, das tierischer (beispielsweise aus Krill oder Eigelb) oder pflanzlicher (beispielsweise Sojalecithin) Herkunft sein kann. Der erfindungsgemäß am meisten bevorzugte Emulgator ist Eilecithin.

Das Eilecithin wird vorzugsweise in Konzentrationen von 0,3 bis 2,5 %, vorzugsweise in Konzentrationen von 0,6 bis 1,5 % bezogen auf das Gesamtgewicht der Emulsion eingesetzt.

### Der Co-Emulgator

Das erfindungsgemäße Verfahren kann die Zugabe zumindest eines Co-Emulgators umfassen. Der Begriff "Co-Emulgator" bezieht sich auf amphiphile Substanzen, die die Emulsion durch Reduktion der Grenzflächenspannung zwischen der Öl- und der Wasserphase stabilisieren und zusammen mit dem Emulgator an der Phasengrenze akkumulieren. Anders als der Emulgator muss der Co-Emulgator allein nicht geeignet sein, selbst-assoziierte Strukturen wie beispielsweise Mizellen zu bilden. Üblicherweise wird der Co-Emulgator in geringeren Konzentrationen eingesetzt als der Emulgator.

Geeignete Co-Emulgatoren sind beispielsweise gesättigte und ungesättigte Fettsäuren und deren Salze.

Sie werden in Konzentrationen von 0,005 bis 1 % bezogen auf das Gesamtgewicht der Emulsion eingesetzt.

Vorzugsweise ist der Co-Emulgator eine ungesättigte, vorzugsweise eine einfach ungesättigte, langkettige Fettsäure oder ein Alkalisalz davon, am meisten bevorzugt Ölsäure oder Natrium-Oleat. Die eingesetzte Menge an Co-Emulgator liegt vorzugsweise zwischen 0,01 und 1 %, insbesondere bevorzugt zwischen 0,02 und 0,5 % bezogen auf das Gesamtgewicht der Emulsion.

### Das Co-Solvens

Das erfindungsgemäße Verfahren kann die Zugabe zumindest eines Co-Solvens umfassen. Der Begriff "Co-Solvens" bezieht sich auf Moleküle, die die Stabilität der nach dem erfindungsgemäßen Verfahren hergestellten Emulsionen verbessern können. Sie reduzieren die Dielektrizitätskonstante des Wassers und machen dessen Umgebung hydrophober. Außerdem erhöhen Co-Solventien die Menge an molekular dispergiertem Emulgator in der Wasserphase. Die Verfügbarkeit von freiem Emulgator unterstützt die Solubilisierung hydrophober Moleküle.

Geeignete Co-Solventien sind beispielsweise Ethanol, Propylenglykol (1,2-Propandiol), Polyethylenglykole (PEG) mit einem Molekulargewicht von 100 bis 20.000 Gramm pro Mol und Polypropylenglykole (PPG) mit einem Molekulargewicht von 180 bis 7000 Gramm pro Mol.

Sie werden in Konzentrationen von 0,1 bis 2,0 %, vorzugsweise 0,70 bis 1,40 %, insbesondere bevorzugt 0,80 bis 1,30 %, und am meisten bevorzugt 0,90 bis 1,20 % bezogen auf das Gesamtgewicht der Emulsion eingesetzt.

Sie werden vorzugsweise in Schritt e) des erfindungsgemäßen Verfahrens zugesetzt.

Vorzugsweise ist das Co-Solvens ein PEG, insbesondere vorzugsweise PEG 200 oder PEG 400.

Die eingesetzte Menge an PEG liegt vorzugsweise zwischen 0,7 und 1,4 %, insbesondere bevorzugt zwischen 0,9 und 1,2 % bezogen auf das Gesamtgewicht der Emulsion.

### Das Isotonisierungsmittel

Das erfindungsgemäße Verfahren kann die Zugabe zumindest eines pharmazeutisch verträglichen Isotonisierungsmittels umfassen.

Geeignete Isotonisierungsmittel sind Salze (beispielsweise Natriumchlorid), Polyole (beispielsweise Mannitol oder Glycerin) und Zucker (beispielsweise Lactose oder Glucose).

Sie werden in Konzentrationen von 0,1 bis 10 %, vorzugsweise 0,5 bis 5 %, insbesondere vorzugsweise von 0,7 bis 3 % bezogen auf das Gesamtgewicht der Emulsion eingesetzt.

Vorzugsweise ist das Isotonisierungsmittel ein Polyol, insbesondere vorzugsweise Glycerin.

Vorzugsweise wird das Glycerin in Mengen von 1 bis 5 %, insbesondere bevorzugt 1 bis 3 %, am meisten bevorzugt 2 bis 2,5 % bezogen auf das Gesamtgewicht der Emulsion eingesetzt.

Die Osmolalität der nach dem erfindungsgemäßen Verfahren hergestellten Emulsionen liegt vorzugsweise zwischen 305 und 420 mOsmol/kg gemessen mit einem Dampfdruck Osmometer, Modell 5520 (Vapro^{™}) nach USP 785.

### Das Antioxidans

Das erfindungsgemäße Verfahren kann die Zugabe zumindest eines Antioxidans umfassen. Das Antioxidans kann jede pharmazeutische verträgliche Substanz mit antioxidativer Wirkung sein. Beispiele für geeignete Antioxidanzien sind Natriummetasulfit, Natriumbisulfit, Natriumsulfit, Natriumthiosulfat, Thioglycerol, Thiosorbitol, Thioglycolsäure, Cystein (vorzugsweise als Cysteinhydrochlorid), N-Acetyl-Cystein, Zitronensäure, alpha-Tocopherol, beta-Tocopherol, gamma-Tocopherol, hydrophile Derivate von Vitamin E, lipophile Derivate von Vitamin E (z.B. Vitamin-E-Acetat), butyliertes Hydroxyanisol (BHA), butyliertes Hydroxytoluol (BHT), t-butyl-Hydrochinon (TBHQ), Monothioglycerin, Propylgallat, Histidin, Coenzyme Q10, Tocotrienole, Carotenoide, Chinone, Bioflavonoide, Polyphenole, Ascorbinsäure (Vitamin C) und Ascorbinsäurederivate (z.B. Ascorbylpalmitat, Isoascorbinsäure) und Harnsäure.

Das Antioxidans wird in Konzentrationen von 0,001 bis 0,5 %, vorzugsweise 0,01 bis 0,3 %, bezogen auf das Gesamtgewicht der Emulsion eingesetzt.

Vorzugsweise ist das Antioxidans ausgewählt aus der Gruppe bestehend aus alpha-Tocopherol, beta-Tocopherol, gamma-Tocopherol, und Mischungen davon.

Am meisten bevorzugt ist das Antioxidans alpha-Tocopherol.

Vorzugsweise wird das alpha-Tocopherol in Konzentrationen von 0,01 bis 0,3 %, insbesondere bevorzugt 0,05 bis 0,2 %, bezogen auf das Gesamtgewicht der Emulsion eingesetzt.

### Die Substanz zur Einstellung des pH-Wertes

Das erfindungsgemäße Verfahren kann die Zugabe zumindest einer Substanz zur Einstellung des pH-Wertes umfassen.

Die Substanz zur Einstellung des pH-Wertes kann jede pharmazeutisch geeignete Säure oder Base sein.

Geeignete Säuren sind Zitronensäure, Milchsäure, Phosphorsäure und Salzsäure (HCl).

Vorzugsweise ist die Säure verdünnte Salzsäure, besonders bevorzugt 0,1 M oder 1 M Salzsäure.

Geeignete Basen sind Alkali- und Erdalkalibasen.

Vorzugsweise ist die Base Natriumhydroxid und wird in Form einer wässrigen Lösung (Natronlauge) eingesetzt.

Am meisten bevorzugt ist die Substanz zur Einstellung des pH-Wertes 0,1 M oder 1 M Natronlauge.

### Das Konservierungsmittel

Das erfindungsgemäße Verfahren kann den Zusatz zumindest eines pharmazeutisch verträglichen Konservierungsmittels umfassen.

Geeignete Konservierungsmittel sind p-Hydroxybenzoesäure sowie Derivate und Salze von p-Hydroxybenzoesäure, Sorbinsäure sowie Derivate und Salze von Sorbinsäure, Benzylalkohol, Chlorbutanol, Thiomersal, Chlorhexidin und dessen Salze, Phenylquecksilbersalze, Chlorkresol, Ethylendiamintetraessigsäure und ihre Salze und Phenoxyethanol.

Sie werden in Konzentrationen von 0,001 bis 2,0 % bezogen auf das Gesamtgewicht der Emulsion eingesetzt.

Vorzugsweise ist das Konservierungsmittel Ethylendiamintetraessigsäure (EDTA) oder ein Salz davon und wird in Konzentrationen von 0,05 bis 0,8 %, vorzugsweise von 0,1 bis 0,7 % bezogen auf das Gesamtgewicht der Emulsion eingesetzt.

### Der Behälter

Die nach dem erfindungsgemäßen Verfahren hergestellten Emulsionen werden vor oder nach dem Sterilisieren in einen geeigneten Behälter abgefüllt.

Geeignete Behälter sind Flaschen, Spritzen, Ampullen, Phiolen, Dosen oder Beutel. Sie können aus jedem geeigneten Material, beispielsweise aus Glas, Metall, Verbundmaterialien oder Kunststoff, bestehen und ggf. beschichtet sein, beispielsweise mit Kunststoff oder Silikon.

Vorzugsweise handelt es bei dem geeigneten Behälter um eine Flasche aus Glas oder Kunststoff, eine Spritze aus Glas oder Kunststoff, eine Phiole aus Glas oder Kunststoff oder um einen Kunststoffbeutel.

Auch die Emulsion erhalten in Schritt d) des erfindungsgemäßen Verfahrens kann vor ihrer Weiterverarbeitung in einen geeigneten Behälter abgefüllt werden. Geeignete Behälter umfassen sterile Großpackmittel (IBC; Intermediate Bulk Container), beispielsweise aus Stahl, Edelstahl oder Kunststoff.

### Schritt a)

Das Bereitstellen der Ölphase umfasst das Mischen der verschiedenen Öle, sofern eine Mischung von Ölen verwendet wird, sowie optional die Zugabe zumindest eines Emulgators und/oder zumindest eines Co-Emulgators und/oder zumindest eines Antioxidans.

Das Bereitstellen der Ölphase kann außerdem die Zugabe zumindest eines Arzneistoffs, vorzugsweise eines lipophilen Arzneistoffs, umfassen, der in der Ölphase gelöst, suspendiert oder dispergiert, vorzugsweise gelöst, wird.

Der lipophile Arzneistoff kann beispielsweise Clevidipin, Docetaxel, Paclitaxel, Dexamethason, Diazepam, Cyclosporin, Etomidat, Flurbiprofen, Bupivacain, Amphotericin B oder Propofol sein.

In bevorzugten Ausführungsformen wird der Ölphase Propofol zugesetzt.

Das Bereitstellen der Ölphase kann außerdem die Zugabe mindestens eines Vitamins oder eines Vitaminderivats, vorzugsweise eines lipophilen Vitamins bzw. eines lipophilen Vitaminderivats, umfassen, das in der Ölphase gelöst, suspendiert oder dispergiert, vorzugsweise gelöst, wird. Lipophile Vitamine sind die Vitamine A, D, E und K. Ein lipophiles Vitaminderivat ist beispielsweise Ascorbylpalmitat.

Das Bereitstellen der Ölphase erfolgt vorzugsweise unter Rühren und Erwärmen.

Die Ölphase wird vorzugsweise auf 40 bis 90 °C, vorzugsweise auf 50 bis 80 °C erwärmt.

Wird der Emulgator der Ölphase zusetzt, so wird diese vorzugsweise auf Temperaturen zwischen 70 und 80 °C erwärmt, um das Auflösen/Dispergieren des Emulgators und/oder des Co-Emulgators zu erleichtern/beschleunigen.

Wird der Emulgator der Wasserphase 1 zugesetzt, so wird die Ölphase vorzugsweise auf Temperaturen zwischen 50 und 60 °C erwärmt, damit diese in Schritt c) die gleiche Temperatur aufweist wie die Wasserphase 1.

### Schritt b)

Die Wasserphase 1, bereitgestellt in Schritt b) stellt nicht mehr als 70 %, bevorzugt nicht mehr als 50 %, insbesondere bevorzugt nicht mehr 30 % und am meisten bevorzugt nicht mehr als 20 % der in der Emulsion enthaltenen Gesamtwassermenge bereit.

Vorzugsweise stellt die Wasserphase 1 nicht weniger als 1 %, 2 %, oder 3 %, der in der Emulsion enthaltenen Gesamtwassermenge bereit.

Das Bereitstellen der Wasserphase 1 kann das Mischen des Wassers mit zumindest einem Emulgator und/oder mit zumindest einem Co-Emulgator und/oder mit zumindest einem Konservierungsmittel umfassen.

Es kann außerdem das Einstellen des pH-Wertes, vorzugsweise auf Werte zwischen 6,0 und 10,0, insbesondere zwischen 7,0 und 9,0, besonders bevorzugt zwischen 8,0 und 9,0, umfassen.

Außerdem kann das Bereitstellen der Wasserphase 1 die Zugabe zumindest eines pharmazeutisch verträglichen Isotonisierungsmittels umfassen. Die Konzentration des Isotonisierungsmittels sollte 18 %, vorzugsweise 15 %, insbesondere bevorzugt 14,3 %, bezogen auf das Gesamtgewicht der Wasserphase 1 nicht überschreiten.

Das Bereitstellen der Wasserphase 1 kann außerdem die Zugabe zumindest eines Arzneistoffs, vorzugsweise zumindest eines wasserlöslichen Arzneistoffs, umfassen, der in der Wasserphase 1 gelöst, suspendiert oder dispergiert, vorzugsweise gelöst, wird.

Das Bereitstellen der Wasserphase 1 kann außerdem die Zugabe zumindest eines Vitamins, vorzugsweise zumindest eines wasserlöslichen Vitamins umfassen, das in der Wasserphase 1 gelöst, suspendiert oder dispergiert, vorzugsweise gelöst, wird. Wasserlösliche Vitamine sind die Vitamine B1, B2, B6, B12, Folsäure, Biotin und Vitamin C.

Das Bereitstellen der Wasserphase 1 erfolgt vorzugsweise unter Rühren und Erwärmen. Das Rührwerkzeug kann ein innen- oder außenliegender Hochscherungsmischer sein (z.B. ein Rotor-Stator-System der Firmen IKA oder Ystral).

Die Wasserphase 1 wird vorzugsweise auf 40 bis 90 °C, insbesondere bevorzugt auf 50 bis 80 °C, erwärmt.

Wird der Emulgator der Ölphase zugefügt, so wird die Wasserphase 1 vorzugsweise auf Temperaturen zwischen 70 und 80 °C erwärmt, damit diese in Schritt c) die gleiche Temperatur aufweist wie die Ölphase.

Wird der Emulgator der Wasserphase 1 zugefügt, so wird die Wasserphase 1 vorzugsweise auf Temperaturen zwischen 50 und 60 °C erwärmt, um das Auflösen/Dispergieren des Emulgators und/oder des Co-Emulgators zu erleichtern/beschleunigen.

### Schritt c)

Das Bereitstellen der Prä-Emulsion erfolgt durch Mischen der in Schritt a) bereitgestellten Ölphase mit der in Schritt b) bereitgestellten Wasserphase 1. Das Mischen erfolgt vorzugsweise durch Rühren. Das Rührwerkzeug kann ein innen- oder außenliegender Hochscherungsmischer sein (z.B. ein Rotor-Stator-System der Firmen IKA oder Ystral).

Durch den Eintrag von Scherenergie während der Emulsionsbildung kann sich die Emulsion erwärmen. Die Temperatur wird (beispielsweise über Wärmetauscher) vorzugsweise zwischen 50 bis 65 °C gehalten.

### Schritt d)

Erfindungsgemäß erfolgt die Herstellung der Emulsion in Schritt d) durch Hochdruckhomogenisierung, Ultraschallbehandlung oder mit Hilfe eines Gegenstrahldispergators. Vorzugsweise wird die Emulsion durch Hochdruckhomogenisierung hergestellt.

Die Homogenisierung wird vorzugsweise bei Temperaturen von 40 bis 70 °C, insbesondere bevorzugt von 40 bis 60 °C, am meisten bevorzugt von 50 bis 60 °C durchgeführt.

Die Hochdruckhomogenisierung kann mit Hilfe aller gängigen Hochdruckhomogenisatoren, beispielsweise mit Geräten vom Typ Ariete der Firma GEA, erfolgen.

Vorzugsweise erfolgt das Hochdruckhomogenisieren über mehrere, vorzugsweise 4 bis 6, Zyklen in einem 2-Stufen-Hochdruckhomogenisator, vorzugsweise bei 350 bis 600 bar in Stufe 1 und bei 0 bis 150 bar in Stufe 2.

### Schritt e)

Das Bereitstellen der Wasserphase 2 kann das Mischen des Wassers mit zumindest einem pharmazeutisch verträglichen Isotonisierungsmittel und/oder mit zumindest einer Substanz zur Einstellung des pH-Wertes und/oder mit zumindest einem pharmazeutisch verträglichen Konservierungsmittel und/oder mit zumindest einem Co-Solvens umfassen.

Es kann außerdem das Einstellen des pH-Wertes, vorzugsweise auf Werte zwischen 6,0 und 10,0, insbesondere zwischen 7,0 und 9,0, besonders bevorzugt zwischen 8,0 und 9,0, umfassen.

Der Wasserphase 2 kann außerdem zumindest ein Arzneistoff oder Vitamin, vorzugsweise ein wasserlöslicher Arzneistoff oder ein wasserlösliches Vitamin, zugefügt werden. Der Arzneistoff und/oder das Vitamin werden in der Wasserphase 2 gelöst, suspendiert oder dispergiert, vorzugsweise gelöst. Wasserlösliche Vitamine sind die Vitamine B1, B2, B6, B12, Folsäure, Biotin und Vitamin C.

Das Bereitstellen der Wasserphase 1 erfolgt vorzugsweise unter Rühren. Das Rührwerkzeug kann ein innen- oder außenliegender Hochscherungsmischer sein (z.B. ein Rotor-Stator-System der Firmen IKA oder Ystral).

Die Wasserphase 2 wird vorzugsweise auf 5 bis 25 °C, insbesondere bevorzugt auf 10 bis 20 °C, am meisten bevorzugt auf 10 bis 15 °C temperiert.

### Schritt f)

Das Herstellen der Emulsion in Schritt f) erfolgt durch Mischen der in Schritt d) erhaltenen Emulsion mit der in Schritt e) bereitgestellten Wasserphase 2, vorzugsweise durch Zugabe der Emulsion zur Wasserphase 2, die vorzugsweise in einem geeigneten Tank/Kessel vorgelegt wird.

Das Herstellen der Emulsion erfolgt vorzugsweise unter Rühren mit einem innenliegenden Propellerrührer.

Die Emulsion wird vorzugsweise mit Stickstoff begast, so dass der Sauerstoffgehalt der Emulsion vorzugsweise unter 0,5 mg/L liegt.

### Schritt q)

Das Sterilisieren der Emulsion kann mit allen geeigneten Verfahren, beispielsweise durch Bestrahlen, Autoklavieren oder Begasen, erfolgen.

Das Sterilisieren erfolgt vorzugsweise durch Autoklavieren. Vorzugsweise wird für 8 bis 21 Minuten bei einem Druck von 2 bar und einer Temperatur von 116 bis 123 °C autoklaviert.

Vorzugsweise wird die Emulsion vor dem Autoklavieren in einen der oben genannten geeigneten Behälter abgefüllt.

### BEISPIELE

### Beispiel 1

Es wurde in einer Versuchsreihe bestimmt, bis zu welchem Phasen-Volumen-Verhältnis (Menge der internen Phase - hier also der Ölphase - im Verhältnis zur Summe aus Wasserphase 1 und Ölphase) die Wasserphase 1 reduziert werden kann und welchen Einfluss die Konzentration des Isotonisierungsmittels (hier: Glycerin) hat.

Das in Versuch I durchgeführte Verfahren entspricht dem bekannten Standardverfahren.

**Tabelle 1**

| Versuchsnummer | I | II | III | IV | V | VI | VII |
|---|---|---|---|---|---|---|---|
| Emulsionsbildung | ja | ja | ja | ja | nein | ja | ja |
| Wasserphase 1 | | | | | | | |
| WFI (g) | **75** | **35** | **20** | **15** | **10** | **10** | **10** |
| Glycerin (g) | **2,5** | **2,5** | **2,5** | **2,5** | **2,5** | **0** | **1,5** |
| Eilecithin (g) | 1,2 | 1,2 | 1,2 | 1,2 | 1,2 | 1,2 | 1,2 |
| Natriumoleat (g) | 0,3 | 0,3 | 0,3 | 0,3 | 0,3 | 0,3 | 0,3 |
| 1 M NaOH | q.s. | q.s. | q.s. | q.s. | q.s. | q.s. | q.s. |
| Ölphase | | | | | | | |
| Ölmischung (g) | 20 | 20 | 20 | 20 | 20 | 20 | 20 |
| alpha-Tocopherol (g) | 0,02 | 0,02 | 0,02 | 0,02 | 0,02 | 0,02 | 0,02 |
| Wasserphase 2 | | | | | | | |
| Wasser (g) | 0 | 40 | 55 | 60 | 70 | 70 | 70 |
| Glycerin (g) | 0 | 0 | 0 | 0 | 0 | 2,5 | 1 |
| Gesamtmenge (g) | 100 | 100 | 100 | 100 | 100 | 100 | 100 |
| Phasen-Volumen-Verhältnis | 0,21 | 0,35 | 0,47 | 0,53 | 0,62 | 0,67 | 0,63 |
| Glycerinkonzentration Wasserphase 1 | 3,2 | 6,7 | 11,1 | 14,3 | 20 | 0 | 13 |

Die Öle (eine Mischung aus Sojaöl, MCT, Olivenöl und Fischöl gemäß Beispiel 2 oder 3) wurden auf 55 bis 60°C temperiert, es wurde das Antioxidans (alpha-Tocopherol) zugegeben und für weitere 15 Minuten gerührt.

Parallel dazu wurden jeweils die in Tabelle 1 angegebenen Mengen an Wasser für Injektionszwecke (WFI), die jeweils in der Tabelle angegebenen Mengen Glycerin und die für die Einstellung des pH-Werts auf 8,5 bis 8,75 benötigte Menge Natronlauge in einem weiteren Kessel eingewogen, auf 55 bis 65°C temperiert, und nacheinander wurden der Emulgator (Eilecithin) und der Co-Emulgator (Natriumoleat) zugegeben und in dem WFI dispergiert (Wasserphase 1).

Die erwärmte Ölphase wurde über einen Sterilfilter in die Wasserphase 1 eingezogen und weitere 30 Minuten gemischt.

Das restliche WFI und Glycerin (Wasserphase 2) wurden in den in Tabelle 1 angegebenen Mengen in einem Tank vorgelegt und auf 5 bis 15 °C temperiert.

Die Prä-Emulsion wurde mit einem Microfluidics MF-110F der Firma Microfluidizer hochdruckhomogenisiert (4 Zyklen bei 400/100 bar) und in den Lagertank mit dem WFI bzw. der Polyol-WFI-Mischung gegeben.

Es wurde überraschend gefunden, dass die Wasserphase 1 mindestens bis zu einem Phasen-Volumen-Verhältnis von 0,67 reduziert werden kann (Versuch IV). Bei einer Glycerin-Konzentration von 20 % in der Wasserphase 1 im Homogenisierungsschritt misslang die Emulsionsbildung.

Bei Glycerin-Konzentrationen von bis zu 14,3 % in der Wasserphase 1 im Emulgierschritt (Versuch IV) gelang die Emulsionsbildung. Die Polyol-Konzentration im Emulgierschritt sollte also unter 18 %, vorzugsweise unter 15 % bezogen auf das Gesamtgewicht der Wasserphase 1 liegen.

### Beispiel 2

Unter Verwendung der in Tabelle 2 angegebenen Rohstoffe wurden 157 Chargen einer Emulsion nach dem gängigen Herstellungsverfahren (A) und 156 Chargen nach dem erfindungsgemäßen Verfahren (B) hergestellt.

### Verfahren A)

Das Sojaöl, das MCT-Öl, das Olivenöl und das Fischöl wurden zusammengegeben und auf 55 bis 60 °C temperiert; anschließend wurde das Antioxidans (alpha-Tocopherol) zugegeben und es wurde für weitere 15 Minuten gerührt.

Parallel dazu wurden 240 kg WFI und die zur Einstellung eines pH-Wertes von 8,5 bis 8,75 benötigte Menge Natronlauge in einem weiteren Kessel eingewogen, auf 55 bis 65 °C temperiert, und nacheinander wurden das Glycerin, das Eilecithin und das Natriumoleat zugegeben und in dem WFI dispergiert (Wasserphase 1).

Die erwärmte Ölphase wurde über einen Sterilfilter in die Wasserphase 1 eingezogen und weitere 30 Minuten gemischt.

Die Rohemulsion wurde mit einem Hochdruckhomogenisator vom Typ Rannie 12.51H der Firma APV hochdruckhomogenisiert (4 Zyklen bei 400/100 bar). Anschließend wurden der pH-Wert erneut auf einen Wert zwischen 8,5 bis 8,75 und der Wassergehalt eingestellt.

**Tabelle 2**

| **Rohstoff** | **Menge** (kg) A | **Menge** (kg) B |
|---|---|---|
| Wasser für Injektionszwecke (WFI) | 240,00 | 148,50 |
| Glycerin | 25,00 | 25,00 |
| Eilecithin | 12,00 | 12,00 |
| Natrium Oleat | 0,30 | 0,30 |
| NaOH 1M | q.s. | q.s. |
| Sojaöl | 60,00 | 60,00 |
| MCT-Öl | 60,00 | 60,00 |
| Olivenöl | 50,00 | 50,00 |
| Fischöl | 30,00 | 30,00 |
| alpha-Tocopherol | 0,02 | 0,02 |
| WFI | ad. 1000 | ad. 1000 |
| Stickstoff | q.s. | q.s. |

### Verfahren B)

Das Sojaöl, das MCT-Öl, das Olivenöl und das Fischöl wurden zusammengegeben und auf 55 bis 60 °C temperiert; anschließend wurde das Antioxidans zugegeben und es wurde für weitere 15 Minuten gerührt.

Parallel dazu wurden 148,50 kg WFI und die zur Einstellung eines pH-Wertes von 8,5 bis 8,75 benötigte Menge Natronlauge in einem weiteren Kessel eingewogen, auf 55 bis 65°C temperiert, und nacheinander wurden das Glycerin, das Eilecithin und das Natriumoleat zugegeben und in dem WFI dispergiert (Wasserphase 1).

Die erwärmte Ölphase wurde über einen Sterilfilter in die Wasserphase 1 eingezogen und weitere 30 Minuten gemischt.

Das restliche WFI wurden in einem Tank vorgelegt und auf 5 bis 15 °C temperiert.

Die Prä-Emulsion wurde mit einem Hochdruckhomogenisator vom Typ Rannie 12.51H der Firma APV hochdruckhomogenisiert (4 Zyklen bei 400/100 bar) und in den Lagertank mit der Glycerin-WFI-Mischung gegeben. Anschließend wurden der pH-Wert und der Wassergehalt eingestellt.

Es wurde für jede Charge der PFATs-Wert ermittelt.

Es ergeben sich die folgenden Mittelwerte und Standardabweichungen:
Durchschnittlicher PFATs (Verfahren A, n = 157): 0,035 ± 0,021
Durchschnittlicher PFATs (Verfahren B, n = 156): 0,006 ± 0,004

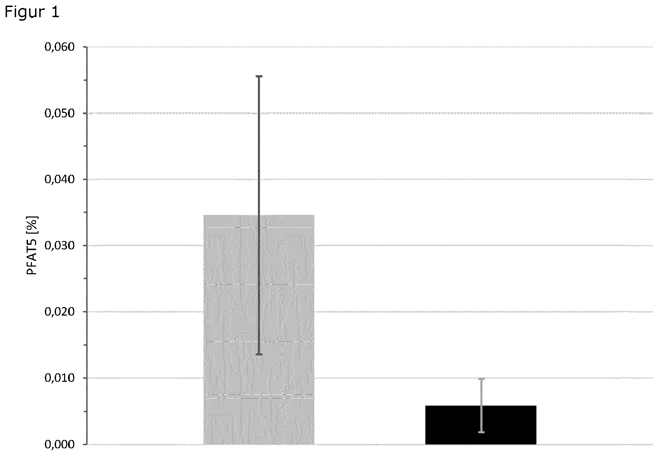

Das erfindungsgemäße Verfahren hat den durchschnittlichen PFATs-Wert also erheblich verringert (siehe auch Figur 1).

Keine der nach dem erfindungsgemäßen Verfahren hergestellten Chargen musste vernichtet werden, weil sie einen PFATs-Wert von mehr als 0,05 % aufwies. Insbesondere wies keine der nach dem erfindungsgemäßen Verfahren hergestellten Chargen einen PFATs-Wert von mehr als 0,017 % auf.

Die mittleren Tröpfchendurchmesser (D₅₀) unterschieden sich nicht. Jedoch führt das erfindungsgemäße Verfahren B zu geringeren Abweichungen beim mittleren Tröpfchendurchmesser.

Die folgenden Tröpfchengrößen wurden gemessen (angegeben sind die Mittelwerte und Standardabweichungen):
D₅₀ (Verfahren A): 351 nm ± 18 nm
D₅₀ (Verfahren B): 353 nm ± 6 nm

### Beispiel 3

**Tabelle 3**

| **Rohstoff** | **Menge** (kg) |
|---|---|
| Wasser für Injektionszwecke (WFI) | 115,00 |
| Glycerin | 25,00 |
| Eilecithin | 12,00 |
| Natrium Oleat | 0,30 |
| NaOH 1M | q.s. |
| Sojaöl | 60,00 |
| MCT-Öl | 60,00 |
| Olivenöl | 50,00 |
| Fischöl | 30,00 |
| alpha-Tocopherol | 0,02 |
| WFI | ad. 1000 |
| Stickstoff | q.s. |

Das Sojaöl, das MCT-Öl, das Olivenöl und das Fischöl wurden zusammengegeben und auf 55 bis 60 °C temperiert; anschließend wurde das Antioxidans zugegeben und es wurde für weitere 15 Minuten gerührt.

Parallel dazu wurden 115 kg WFI und die zur Einstellung eines pH-Wertes von 8,5 bis 8,75 benötigte Menge Natronlauge in einem weiteren Kessel eingewogen, auf 55 bis 65°C temperiert, und nacheinander wurden das Eilecithin und das Natriumoleat zugegeben und in dem WFI dispergiert (Wasserphase 1).

Die erwärmte Ölphase wurde über einen Sterilfilter in die Wasserphase 1 eingezogen und weitere 30 Minuten gemischt.

Das Glycerin und das restliche WFI (Wasserphase 2) wurden in einem Tank vorgelegt und auf 5 bis 15 °C temperiert.

Die Prä-Emulsion wurde mit einem Hochdruckhomogenisator vom Typ Rannie 12.51H der Firma APV hochdruckhomogenisiert (4 Zyklen bei 400/100 bar) und in den Lagertank mit der Glycerin-WFI-Mischung gegeben.

Anschließend wurden der pH-Wert und der Wassergehalt eingestellt.

Unmittelbar nach der Herstellung lag der mittlere Tröpfchendurchmesser bei 360 nm (D₅₀), der PFATs-Wert bei 0,006 % und der Gehalt an nicht veresterten Fettsäuren (NEFA) bei 2,0 mEq/L.

### Beispiel 4

**Tabelle 4**

| **Rohstoff** | **Menge** (g) |
|---|---|
| Wasser für Injektionszwecke (WFI) | 99,00 |
| Glycerin | 25,00 |
| Eilecithin | 12,00 |
| Ölsäure | 0,40 |
| NaOH 1M | q.s. |
| Sojaöl | 100,00 |
| MCT-Öl | 100,00 |
| Propofol | 10,00 |
| WFI | ad. 1000 |
| Stickstoff | q.s. |

Sojaöl, MCT-Öl, die Ölsäure und Propofol wurden zusammengegeben, auf 55 bis 65 °C temperiert und 15 Minuten gerührt.

Parallel dazu wurden 99 kg WFI und die zur Einstellung eines pH-Wertes von 8,0 bis 8,75 benötigte Menge Natronlauge in einem weiteren Kessel eingewogen, auf 55 bis 65°C temperiert, das Eilecithin zugegeben und in dem WFI dispergiert (Wasserphase 1).

Die erwärmte Ölphase wurde über einen Sterilfilter in die Wasserphase 1 eingezogen und weitere 30 Minuten gemischt.

Das Glycerin und das restliche WFI (Wasserphase 2) wurden in einem Tank vorgelegt und auf 5 bis 15°C temperiert.

Die Prä-Emulsion wurde mit einem Hochdruckhomogenisator vom Typ Microfluidics MF-110F der Firma Microfluidizer hochdruckhomogenisiert (4 Zyklen 400/100 bar) und in den Lagertank mit der Glycerin-WFI-Mischung gegeben.

Anschließend wurden der pH-Wert (auf 8,2) und der Wassergehalt eingestellt. Der mittlere Tröpfchendurchmesser lag bei 236 nm (D₅₀).

### Beispiel 5

**Tabelle 5**

| **Rohstoff** | **Menge** (kg) |
|---|---|
| Wasser für Injektionszwecke (WFI) | 76,00 |
| EDTA | 0,06 |
| Glycerin | 22,50 |
| Natriumoleat | 0,30 |
| Eilecithin | 12,00 |
| NaOH 1M | q.s. |
| Sojaöl | 100,00 |
| Propofol | 10,00 |
| WFI | ad. 1000 |
| Stickstoff | q.s. |

Das Soja-Öl wurde auf 73 bis 77°C temperiert. Es wurde das Eilecithin portionsweise zugegeben und weitere 15 Minuten gerührt bis das komplette Eilecithin gelöst war.

Parallel dazu wurden 76 kg WFI und die zur Einstellung eines pH-Wertes von 8,5 bis 9,5 benötigte Menge Natronlauge in einem weiteren Kessel eingewogen und auf 73 bis 77 °C temperiert. Dann wurden nacheinander das EDTA sowie das Natriumoleat zugegeben und gelöst (Wasserphase 1).

Die erwärmte Ölphase wurde über einen Sterilfilter in die Wasserphase 1 eingezogen und weitere 30 Minuten gemischt.

Das Glycerin und das restliche WFI (Wasserphase 2) wurden in einem Tank vorgelegt und auf 5 bis 15°C temperiert.

Die Prä-Emulsion wurde mit einem Hochdruckhomogenisator vom Typ Microfluidics MF-110F der Firma Microfluidizer hochdruckhomogenisiert (6 Zyklen 490/0 bar) und in den Lagertank mit der Glycerin-WFI-Mischung gegeben.

Anschließend wurden der Wassergehalt und der pH-Wert eingestellt.

Der mittlere Tröpfchendurchmesser lag bei 267 nm (D₅₀).

### Beispiel 6

**Tabelle 6**

| **Rohstoff** | **Menge** (kg) |
|---|---|
| Wasser für Injektionszwecke (WFI) | 140,00 |
| Glycerin | 22,00 |
| Natriumoleat | 0,30 |
| Eilecithin | 12,00 |
| NaOH 1M | q.s. |
| Sojaöl | 200,00 |
| WFI | ad. 1000 |
| Stickstoff | q.s. |

Das Soja-Öl wurde auf 73 bis 77°C temperiert. Es wurde das Eilecithin portionsweise zugegeben und weitere 15 Minuten gerührt bis das komplette Eilecithin gelöst war.

Parallel dazu wurden 140 kg WFI, 0,30 kg Natriumoleat und die zur Einstellung eines pH-Wertes von 8,5 bis 9,5 benötigte Menge Natronlauge in einem weiteren Kessel eingewogen und auf 73 bis 77°C temperiert (Wasserphase 1).

Die erwärmte Ölphase wurde über einen Sterilfilter in die Wasserphase 1 eingezogen und weitere 30 Minuten gemischt.

Das Glycerin und das restliche WFI (Wasserphase 2) wurden in einem Tank vorgelegt und auf 5 bis 15°C temperiert.

Die Prä-Emulsion wurde mit einem Hochdruckhomogenisator vom Typ Microfluidics MF-110F der Firma Microfluidizer hochdruckhomogenisiert (6 Zyklen 560/120 bar) und in den Lagertank mit der Glycerin-WFI-Mischung gegeben.

Anschließend wurden der Wassergehalt und der pH-Wert eingestellt.

Der mittlere Tröpfchendurchmesser lag bei 393 nm (D₅₀).

### BESCHREIBUNG DER ABBILDUNG

Figur 1 zeigt die Mittelwerte (mit Standardabweichungen) der PFATs-Werte (ermittelt nach USP 729, Methode 2) der nach Beispiel 2 erhaltenen Emulsionschargen. Dabei stellt der graue Balken den Mittelwert der PFATs-Werte der nach Verfahren A hergestellten Chargen dar, während der schwarze Balken für den Mittelwert der PFATs-Werte der nach dem erfindungsgemäßen Verfahren B hergestellten Chargen steht.

## Patentansprüche

1. Verfahren zur Herstellung einer Öl-in-Wasser-Emulsion umfassend eine Wasserphase und 1 bis 40 %, bevorzugt 5 bis 30 %, am meisten bevorzugt 10 bis 30 % einer Ölphase bezogen auf das Gesamtgewicht der Emulsion, wobei das Verfahren die folgenden Schritte umfasst
a) Bereitstellen einer Ölphase umfassend eines oder mehrere Öle, ausgewählt aus der Gruppe bestehend aus Fischöl, Fischölextrakt, Krillöl, mikrobiell erzeugten Ölen, Algen-Ölen, Pilzölen, und Pflanzenölen, und optional ein pharmazeutisch verträgliches Antioxidans und/oder zumindest einen pharmazeutisch verträglichen Co-Emulgator,
b) Bereitstellen einer Wasserphase 1 umfassend Wasser und optional zumindest einen pharmazeutisch verträglichen Co-Emulgator und/oder zumindest eine Substanz zur Einstellung des pH-Wertes und/oder zumindest ein pharmazeutisch verträgliches Konservierungsmittel und/oder zumindest ein pharmazeutisch verträgliches Isotonisierungsmittel, wobei das Isotonisierungsmittel in einer Konzentration von höchstens 18 %, bezogen auf das Gesamtgewicht der ersten Wasserphase vorliegt,
c) Herstellen einer Prä-Emulsion durch Mischen der Ölphase, bereitgestellt in Schritt a), mit der Wasserphase 1, bereitgestellt in Schritt b),
d) Herstellen einer ersten Emulsion durch Homogenisieren der Prä-Emulsion, bereitgestellt in Schritt c),
e) Bereitstellen einer Wasserphase 2 umfassend Wasser und optional zumindest ein pharmazeutisch verträgliches Isotonisierungsmittel und/oder zumindest eine Substanz zur Einstellung des pH-Wertes und/oder zumindest ein pharmazeutisch verträgliches Konservierungsmittel,
f) Herstellen der Emulsion durch Mischen der ersten Emulsion, bereitgestellt in Schritt d), mit der Wasserphase 2, bereitgestellt in Schritt e) und
g) Sterilisieren der Emulsion, erhalten in Schritt f),
wobei die Emulsion vor oder nach dem Sterilisieren in einen geeigneten Behälter gefüllt wird,
wobei in Schritt a) und/oder in Schritt b) zumindest ein pharmazeutisch verträglicher Emulgator zugesetzt wird, und
wobei die Wasserphase 1, bereitgestellt in Schritt b), nicht mehr als 30 %, vorzugsweise nicht mehr als 20 %, der in der Emulsion enthaltenen Gesamtwassermenge bereitstellt.

2. Verfahren nach Anspruch 1, wobei die Emulsion zur parenteralen Verabreichung vorgesehen ist und wobei das Wasser, das bei der Bereitstellung der Wasserphasen 1 und 2 in den Schritten b) und e) verwendet wird, Wasser für Injektionszwecke ist.

3. Verfahren nach Anspruch 1 oder 2, wobei der pharmazeutisch verträgliche Emulgator in einer Konzentration von 0,1 bis 5 % bezogen auf das Gesamtgewicht der Emulsion zugesetzt wird und wobei der pharmazeutisch verträgliche Emulgator vorzugsweise Lecithin ist.

4. Verfahren nach einem der vorangehenden Ansprüche, wobei der pharmazeutisch verträgliche Co-Emulgator Natrium-Oleat ist und in Schritt b) zugefügt wird oder wobei der pharmazeutisch verträgliche Co-Emulgator Ölsäure ist und in Schritt a) zugefügt wird.

5. Verfahren nach einem der vorangehenden Ansprüche, wobei die Öl-in-Wasser-Emulsion ein pharmazeutisch verträgliches Isotonisierungsmittel umfasst, wobei das pharmazeutisch verträgliche Isotonisierungsmittel vorzugsweise ein Polyol, besonders bevorzugt Glycerin, ist.

6. Verfahren nach einem der vorangehenden Ansprüche, wobei das pharmazeutisch verträgliche Isotonisierungsmittel in Schritt b) zugesetzt wird und wobei das pharmazeutisch verträgliche Isotonisierungsmittel in Schritt b) in einer Konzentration von höchstens 15 %, vorzugsweise höchstens 14,3 % bezogen auf das Gesamtgewicht der Wasserphase 1 vorliegt.

7. Verfahren nach einem der Ansprüche 1 bis 5, wobei das Isotonisierungsmittel ausschließlich in Schritt e) zugefügt wird.

8. Verfahren nach einem der vorangehenden Ansprüche, wobei die Emulsion 10 % oder 20 % einer Ölphase bezogen auf das Gesamtgewicht der Emulsion umfasst.

9. Verfahren nach einem der vorangehenden Ansprüche, wobei die Ölphase ein Pflanzenöl und/oder ein oder mehrere Öle, ausgewählt aus der Gruppe bestehend aus Fischöl, Fischölextrakt und Krillöl umfasst.

10. Verfahren nach einem der vorangehenden Ansprüche, wobei die Ölphase Sojaöl, mittelkettige Triglyceride, Olivenöl, Fischöl, Fischölextrakt oder Mischungen davon, vorzugsweise Sojaöl, mittelkettige Triglyceride, Olivenöl und Fischöl, umfasst.

11. Verfahren nach einem der vorangehenden Ansprüche, wobei die Ölphase 25 bis 35 %, vorzugsweise 30 %, Sojaöl, 25 bis 35 %, vorzugsweise 30 %, MCT, 20 bis 30 %, vorzugsweise 25 %, Olivenöl und 10 bis 20 %, vorzugsweise 15 %, Fischöl bezogen auf das Gesamtgewicht der Ölphase umfasst.

12. Verfahren nach einem der vorangehenden Ansprüche, wobei der mittlere Durchmesser der Öltröpfchen in der Emulsion erhalten in Schritt d) und in der Emulsion, erhalten in Schritt f), und nach dem Sterilisieren in Schritt g) zwischen 100 und 500 nm, bevorzugt zwischen 150 und 450 nm liegt.

13. Verfahren nach einem der vorangehenden Ansprüche, wobei der der PFATs-Wert der Emulsion erhalten in Schritt d) und in der Emulsion, erhalten in Schritt f), vor und nach dem Sterilisieren in Schritt g) unter 0,05 %, vorzugsweise unter 0,04 %, besonders bevorzugt unter 0,03 % und am meisten bevorzugt unter 0,02 % liegt.

14. Verfahren nach einem der vorangehenden Ansprüche, wobei der durchschnittliche PFATs-Wert der Emulsion erhalten in Schritt d) und in der Emulsion, erhalten in Schritt f), vor und nach dem Sterilisieren in Schritt g) unter 0,035 %, vorzugsweise unter 0,030 %, besonders bevorzugt unter 0,025 %, stärker bevorzugt unter 0,020 %, noch stärker bevorzugt unter 0,015 % und am meisten bevorzugt unter 0,010 % liegt.

15. Verfahren nach einem der vorangehenden Ansprüche, wobei die Emulsion vor dem Sterilisieren in einen geeigneten Behälter, vorzugsweise in eine Glasflasche, eine Kunststoffspritze oder einen Kunststoffbeutel, abgefüllt wird und wobei das Sterilisieren vorzugsweise durch Autoklavieren erfolgt.

16. Öl-in-Wasser-Emulsion umfassend eine Wasserphase und 1 bis 40 %, bevorzugt 5 bis 30 %, am meisten bevorzugt 10 bis 30 %, einer Ölphase bezogen auf das Gesamtgewicht der Emulsion, erhalten nach dem Verfahren gemäß einem der Ansprüche 1 bis 15.

17. Öl-in-Wasser-Emulsion nach Anspruch 16 zur Verwendung als Medikament oder zur Verwendung in der Bereitstellung parenteraler Ernährung.

18. Öl-in-Wasser-Emulsion, erhalten in Schritt d) des Verfahrens nach einem der Ansprüche 1 bis 15.

## Claims

1. A method for preparing an oil-in-water emulsion comprising a water phase and 1 to 40%, preferably 5 to 30%, most preferably 10 to 30%, of an oil phase based on the total weight of the emulsion, wherein the method comprises the following steps:
a) Providing an oil phase comprising one or more oils selected from the group consisting of fish oil, fish oil extract, krill oil, microbially produced oils, algae oils, fungal oils, and vegetable oils, and optionally a pharmaceutically acceptable antioxidant and/or at least one pharmaceutically acceptable co-emulsifier,
b) Providing a water phase 1 comprising water and optionally at least one pharmaceutically acceptable co-emulsifier and/or at least one substance for adjusting the pH value and/or at least one pharmaceutically acceptable preservative and/or at least one pharmaceutically acceptable isotonic agent, wherein the isotonic agent is present in a concentration of at most 18%, based on the total weight of the first water phase,
c) Preparing a pre-emulsion by mixing the oil phase provided in step a) with the water phase 1 provided in step b),
d) Preparing a first emulsion by homogenizing the pre-emulsion provided in step c),
e) Providing a water phase 2 comprising water and optionally at least one pharmaceutically acceptable isotonic agent and/or at least one substance for adjusting the pH value and/or at least one pharmaceutically acceptable preservative,
f) Preparing the emulsion by mixing the first emulsion provided in step d) with the water phase 2 provided in step e) and
g) Sterilizing the emulsion obtained in step f),
wherein the emulsion is filled into a suitable container before or after sterilization,
wherein in step a) and/or in step b) at least one pharmaceutically acceptable emulsifier is added, and
wherein the water phase 1 provided in step b) provides not more than 30%, preferably not more than 20%, of the total amount of water contained in the emulsion.

2. The method according to claim 1, wherein the emulsion is intended for parenteral administration and wherein the water used in providing the water phases 1 and 2 in steps b) and e) is water intended for injection.

3. The method according to claim 1 or 2, wherein the pharmaceutically acceptable emulsifier is added in a concentration of 0.1 to 5% based on the total weight of the emulsion and wherein the pharmaceutically acceptable emulsifier is preferably lecithin.

4. The method according to one of the preceding claims, wherein the pharmaceutically acceptable co-emulsifier is sodium oleate and is added in step b) or wherein the pharmaceutically acceptable co-emulsifier is oleic acid and is added in step a).

5. The method according to one of the preceding claims, wherein the oil-in-water emulsion comprises a pharmaceutically acceptable isotonic agent, wherein the pharmaceutically acceptable isotonic agent is preferably a polyol, particularly preferably glycerin.

6. The method according to one of the preceding claims, wherein the pharmaceutically acceptable isotonic agent is added in step b) and wherein the pharmaceutically acceptable isotonic agent in step b) is present in a concentration of at most 15%, preferably at most 14.3%, based on the total weight of the water phase 1.

7. The method according to one of claims 1 to 5, wherein the isotonic agent is added exclusively in step e).

8. The method according to one of the preceding claims, wherein the emulsion comprises 10% or 20% of an oil phase based on the total weight of the emulsion.

9. The method according to one of the preceding claims, wherein the oil phase comprises a vegetable oil and/or one or more oils selected from the group consisting of fish oil, fish oil extract and krill oil.

10. The method according to one of the preceding claims, wherein the oil phase comprises soybean oil, medium chain triglycerides, olive oil, fish oil, fish oil extract or mixtures thereof, preferably soybean oil, medium chain triglycerides, olive oil and fish oil.

11. The method according to one of the preceding claims, wherein the oil phase comprises 25 to 35%, preferably 30%, soybean oil, 25 to 35%, preferably 30%, MCT, 20 to 30%, preferably 25%, olive oil and 10 to 20%, preferably 15%, fish oil based on the total weight of the oil phase.

12. The method according to one of the preceding claims, wherein the average diameter of the oil droplets in the emulsion obtained in step d) and in the emulsion obtained in step f), and after sterilization as per step g), is between 100 and 500 nm, preferably between 150 and 450 nm.

13. The method according to one of the preceding claims, wherein the PFAT₅-value of the emulsion obtained in step d) and in the emulsion obtained in step fj, before and after sterilization as per step g), is below 0.05%, preferably below 0.04%, particularly preferably below 0.03% and most preferably below 0.02%.

14. The method according to one of the preceding claims, wherein the average PFAT₅-value of the emulsion obtained in step d) and in the emulsion obtained in step fj, before and after sterilization as per step g), is below 0.035%, preferably below 0.030%, particularly preferably below 0.025%, more preferably below 0.020%, even more preferably below 0.015% and most preferably below 0.010%.

15. The method according to one of the preceding claims, wherein the emulsion is loaded into a suitable container, preferably into a glass bottle, a plastic syringe or a plastic bag, before sterilization and wherein sterilization is preferably carried out by autoclaving.

16. An oil-in-water emulsion comprising a water phase and 1 to 40%, preferably 5 to 30%, most preferably 10 to 30%, of an oil phase based on the total weight of the emulsion, obtained by the method according to one of claims 1 to 15.

17. The oil-in-water emulsion according to claim 16 for use as a medicament or for use in providing parenteral nutrition.

18. An oil-in-water emulsion obtained in step d) of the method according to one of claims 1 to 15.

## Revendications

1. Procédé de préparation d'une émulsion huile dans eau comprenant une phase aqueuse et 1 à 40 %, préférablement 5 à 30 %, plus préférablement 10 à 30 % d'une phase huileuse par rapport au poids total de l'émulsion, le procédé comprenant les étapes suivantes :
a) Fournir une phase huileuse comprenant un ou plusieurs types d'huiles, sélectionnées parmi le groupe constitué d'huile de poisson, d'extrait d'huile de poisson, d'huile de krill, d'huiles microbiennes, d'huiles d'algues, d'huiles de champignons et d'huiles végétales, et éventuellement un antioxydant pharmaceutiquement acceptable et/ou au moins un co-émulsifiant pharmaceutiquement acceptable,
b) Fournir une phase aqueuse 1 comprenant de l'eau et éventuellement au moins un co-émulsifiant pharmaceutiquement acceptable et/ou au moins une substance pour le réglage du pH et/ou au moins un conservateur pharmaceutiquement acceptable et/ou au moins un agent d'isotonisation pharmaceutiquement acceptable, l'agent d'isotonisation étant présent à une concentration maximale de 18 % par rapport au poids total de la première phase aqueuse,
c) Préparer une pré-émulsion en combinant la phase huileuse, fournie à l'étape a), avec la phase aqueuse 1, fournie à l'étape b),
d) Préparer une première émulsion par homogénéisation de la pré-émulsion fournie à l'étape c),
e) Fournir une phase aqueuse 2 comprenant de l'eau et, en option, au moins un agent isotonisant pharmaceutiquement acceptable et/ou au moins une substance pour ajuster le pH et/ou au moins un autre conservateur pharmaceutiquement acceptable,
f) Préparer l'émulsion en combinant la première émulsion fournie à l'étape d) avec la phase aqueuse 2 fournie à l'étape e) et
g) Stériliser l'émulsion obtenue à l'étape f),
l'émulsion étant versée dans un récipient approprié avant ou après stérilisation,
avec ajout d'au moins un émulsifiant pharmaceutiquement acceptable à l'étape a) et/ou à l'étape b), et
dans laquelle la phase aqueuse 1, fournie à l'étape b), fournit au plus 30 %, préférablement au plus 20 %, de la quantité totale d'eau présente dans l'émulsion.

2. Procédé selon la revendication 1, dans lequel l'émulsion est destinée à une administration parentérale et dans lequel l'eau utilisée pour fournir les phases aqueuses 1 et 2 aux étapes b) et e) est de l'eau destinée à des injections.

3. Procédé selon la revendication 1 ou 2, dans lequel l'émulsifiant pharmaceutiquement acceptable est ajouté à une concentration de 0,1 à 5 % par rapport au poids total de l'émulsion, et dans lequel l'émulsifiant pharmaceutiquement acceptable est de préférence de la lécithine.

4. Procédé selon l'une des revendications précédentes, dans lequel le co-émulsifiant pharmaceutiquement acceptable est de l'oléate de sodium et est ajouté à l'étape b), ou dans lequel le co-émulsifiant pharmaceutiquement acceptable est de l'acide oléique et est ajouté à l'étape a).

5. Procédé selon l'une des revendications précédentes, dans lequel l'émulsion huile dans eau comprend un agent isotonisant pharmaceutiquement acceptable, l'agent isotonisant étant de préférence un polyol, et plus particulièrement de la glycérine.

6. Procédé selon l'une des revendications précédentes, dans lequel l'agent isotonisant pharmaceutiquement acceptable est ajouté à l'étape b), et dans lequel cet agent isotonisant est présent à une concentration maximale de 15 %, préférablement au maximum de 14,3 %, par rapport au poids total de la phase aqueuse 1.

7. Procédé selon l'une des revendications 1 à 5, dans lequel l'agent isotonisant est ajouté uniquement à l'étape e).

8. Procédé selon l'une des revendications précédentes, dans lequel l'émulsion comprend 10 % ou 20 % de phase huileuse par rapport au poids total de l'émulsion.

9. Procédé selon l'une des revendications précédentes, dans lequel la phase huileuse comprend une huile végétale et/ou un ou plusieurs huiles sélectionnées parmi le groupe constitué d'huile de poisson, d'extrait d'huile de poisson et d'huile de krill.

10. Procédé selon l'une des revendications précédentes, dans lequel la phase huileuse comprend de l'huile de soja, des triglycérides à chaîne moyenne, de l'huile d'olive, de l'huile de poisson, de l'extrait d'huile de poisson ou des mélanges de ces composants, de préférence de l'huile de soja, des triglycérides à chaîne moyenne, de l'huile d'olive et de l'huile de poisson.

11. Procédé selon l'une des revendications précédentes, dans lequel la phase huileuse comprend 25 à 35 %, de préférence 30 %, d'huile de soja, 25 à 35 %, de préférence 30 %, de MCT, 20 à 30 %, de préférence 25 %, d'huile d'olive et 10 à 20 %, de préférence 15 %, d'huile de poisson, par rapport au poids total de la phase huileuse.

12. Procédé selon l'une des revendications précédentes, dans lequel le diamètre moyen des gouttelettes d'huile dans l'émulsion obtenue à l'étape d) et dans l'émulsion obtenue à l'étape f), ainsi qu'après stérilisation à l'étape g), se situe entre 100 et 500 nm, de préférence entre 150 et 450 nm.

13. Procédé selon l'une des revendications précédentes, dans lequel la valeur PFAT₅ de l'émulsion obtenue à l'étape d) et dans l'émulsion obtenue à l'étape f), ainsi qu'avant et après la stérilisation à l'étape g), est inférieure à 0,05 %, de préférence inférieure à 0,04 %, particulièrement inférieure à 0,03 %, et idéalement inférieure à 0,02 %.

14. Procédé selon l'une des revendications précédentes, dans lequel la valeur PFAT₅ moyenne de l'émulsion obtenue à l'étape d) et dans l'émulsion obtenue à l'étape f), ainsi qu'avant et après la stérilisation à l'étape g), est inférieure à 0,035 %, préférablement inférieure à 0,030 %, particulièrement inférieure à 0,025 %, plus préférablement inférieure à 0,020 %, encore plus préférablement inférieure à 0,015 %, et idéalement inférieure à 0,010 %.

15. Procédé selon l'une des revendications précédentes, dans lequel l'émulsion est déversée dans un récipient approprié, de préférence une bouteille en verre, une seringue en plastique ou un sac en plastique, avant la stérilisation, et la stérilisation est réalisée de préférence par autoclave.

16. Émulsion huile dans eau comprenant une phase aqueuse et une phase huileuse de 1 à 40 %, de préférence de 5 à 30 %, idéalement de 10 à 30 %, par rapport au poids total de l'émulsion, obtenue selon le procédé décrit dans l'une des revendications 1 à 15.

17. Émulsion huile dans eau selon la revendication 16 pour une utilisation en tant que médicament ou pour l'administration de nutrition parentérale.

18. Émulsion huile dans eau obtenue à l'étape d) du procédé selon l'une des revendications 1 à 15.
